# EUROPÄISCHE PATENTSCHRIFT

(11) **EP 0 727 416 B1**
(45) Veröffentlichungstag und Bekanntmachung des Hinweises auf die Patenterteilung: **26.01.2000**
(21) Anmeldenummer: 96101976.7
(22) Anmeldetag: 12.02.1996
(51) Int. Cl.: C07C 311/58, C07C 335/42, A61K 31/64

(54) **Substituierte Benzolsulfonylharnstoffe und -thioharnstoffe, Verfahren zu ihrer Herstellung, ihre Verwendung als Medikament oder Diagnostikum sowie sie enthaltendes Medikament**
Substituted benzenesulfonylureas and -thioureas, process for their preparation, their use as medicament or diagnostic agent, as well as medicaments containing them
Benzènesulfonylurées et -thiourées substituées, procédé pour leur préparation, leur utilisation comme médicament ou agent diagnostique, ainsi que médicaments les contenant

(30) Priorität: 17.02.1995 DE 19505397
(43) Veröffentlichungstag der Anmeldung: 21.08.1996
(73) Patentinhaber: HOECHST AKTIENGESELLSCHAFT, 65929 Frankfurt am Main (DE)
(72) Erfinder: Englert, Heinrich Christian, Dr., D-65719 Hofheim (DE); Gerlach, Uwe, Dr., D-65795 Hattersheim (DE); Mania, Dieter, Dr., D-61462 Königstein (DE); Gögelein, Heinz, Dr., D-60259 Frankfurt (DE); Kaiser, Joachim, Dr., D-60431 Frankfurt (DE)

(56) Entgegenhaltungen:
- EP-A- 0 612 724
- DE-B- 1 518 874

## Beschreibung

Die Erfindung betrifft substituierte Benzolsulfonylharnstoffe und -thioharnstoffe der Formel I 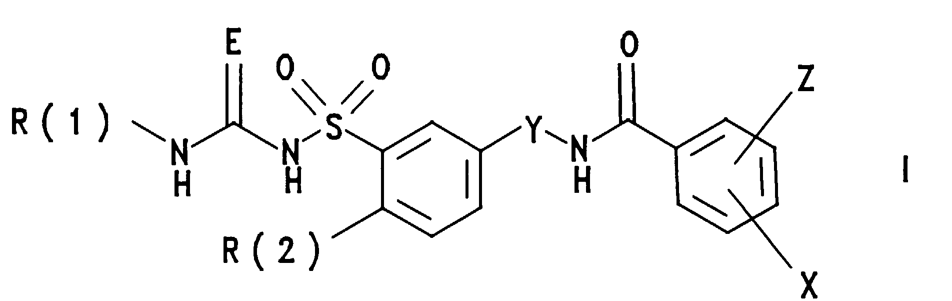 worin bedeuten:
- R(1): Wasserstoff, Methyl oder Trifluormethyl;
- R(2): Alkoxy mit 4, 5, 6, 7, 8, 9 oder 10 C-Atomen, wobei 1 bis 6 Kohlenstoffatome gegen die Heteroatome O, S oder NH ausgetauscht sind;
- E: Sauerstoff oder Schwefel;
- Y: -[CR(3)₂]₁₋₄;
R(3) Wasserstoff oder Alkyl mit 1 oder 2 C-Atomen;
- X: Wasserstoff, Halogen oder Alkyl mit 1, 2, 3, 4, 5 oder 6 C-Atomen;
- Z: Nitro Halogen, Alkoxy mit 1, 2, 3 oder 4 C-Atomen oder Alkyl mit 1, 2, 3 oder 4 C-Atomen;
sowie ihre pharmazeutisch verträglichen Salze.

Sulfonylharnstoffe sind aus der deutschen Offenlegungsschrift 2413514, der deutschen Patentschrift 1 518 874 bekannt. Dort wird deren blutzuckersenkende Wirkung beschrieben. Als Prototyp solcher blutzuckersenkenden Sulfonylharnstoffe gilt das Glibenclamid, das als Mittel zur Behandlung des Diabetes mellitus therapeutisch verwendet wird und in der Forschung ein vielbeachtetes Werkzeug zur Erforschung sogenannter ATP sensitiver Kaliumkanäle dient. Neben seiner blutzuckersenkenden Wirkung besitzt das Glibenclamid noch andere Wirkungen, die bislang therapeutisch noch nicht eingesetzt werden können die aber allesamt auf Blockade eben dieser ATP-sensitiven Kalium-Kanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich da sie den Zustand des Patienten weiter verschlechtern kann.

In der Europäischen Offenlegungsschrift 0 612 724 (HOE 93/F 056) werden bereits Verbindungen mit verminderter Blutzucker-senkender Wirkung beschrieben, die aber für viele Zwecke noch nicht ausreichen. Verbindungen mit einem zweiten Heteroatom im Substituenten R(2) sind dort aber weder vorweggenommen noch nahegelegt.

Aufgabe der vorliegenden Erfindung war es daher, Verbindungen zu synthetisieren, die eine gleich gute Herzwirkung wie Glibenclamid aufweisen, aber den Blutzucker in herzwirksamen Dosen oder Konzentrationen nicht oder deutlich geringer beeinflussen als Glibenclamid.

Dieses Ziel wurde durch die eingangs beschriebenen Verbindungen erreicht.

Bevorzugt sind die Verbindungen I in denen
- R(1): Wasserstoff, Methyl oder Trifluormethyl;
- R(2): Alkoxy mit 4, 5, 6, 7, 8, 9 oder 10 C-Atomen, worin ein bis sechs Kohlenstoffatome gegen die Heteroatome O, NH oder S ausgetauscht sind;
- E: Sauerstoff oder Schwefel;
- Y: ein gerader substituierter oder unsubstituierter Kohlenwasserstoffrest der Formel: -[CR(3)₂)₁₋₄;
R(3) Wasserstoff oder Alkyl mit 1 oder 2 C-Atomen;
- X: Wasserstoff, Chlor, Fluor oder Alkyl mit 1, 2, 3 oder 4 C-Atomen;
- Z: Nitro, Fluor, Chlor, Alkyl mit 1, 2, 3 oder 4 C-Atomen oder Alkoxy mit 1, 2, 3 oder 4 C-Atomen;
sowie ihre pharmazeutisch verträglichen Salze.

Ganz besonders sind die Verbindungen I bevorzugt, in denen bedeuten:
- R(1): Wasserstoff oder Methyl;
- R(2): Alkoxy mit 4, 5, 6, 7, 8, 9 oder 10 C-Atomen, bei der 1 bis 6 Kohlenstoffatome gegen die Heteroatome O, S oder NH ausgetauscht sind;
- E: Sauerstoff oder Schwefel;
- Y: -[CR(3)₂]₁₋₄;
R(3) Wasserstoff oder Alkyl mit 1 oder 2 C-Atomen;
- X: Wasserstoff, Fluor, Chlor oder Alkyl mit 1, 2, 3 oder 4 C-Atomen;
- Z: Chlor, Fluor, Alkyl mit 1, 2, 3 oder 4 C-Atomen oder Alkoxy mit 1, 2, 3 oder 4 C-Atomen;
sowie ihre pharmazeutisch verträglichen Salze.

Insbesondere bevorzugt sind Verbindungen der Formel I, in denen bedeuten:
- R(1): Wasserstoff oder Methyl;
- R(2): Methoxyethoxy oder Methoxyethoxyethoxy;
- E: Sauerstoff oder Schwefel;
- Y: -[CR(3)₂]₂₋₃;
R(3) Wasserstoff oder Methyl;
- X: Wasserstoff, Fluor, Chlor oder Alkyl mit 1, 2 oder 3-C-Atomen;
- Z: Fluor, Chlor, Alkyl mit 1, 2 oder 3-C-Atomen oder Alkoxy mit 1, 2 oder 3-C-Atomen;
sowie ihre pharmazeutisch verträglichen Salze.

Alkyl bedeutet, sofern nicht ausdrücklich anders angegeben, geradkettige, verzweigte oder cyclische gesättigte Kohlenwasserstoffreste mit einem bis sechs Kohlenstoffatomen. Der Terminus Alkoxy steht für einen Ethersubstituenten mit einem geradkettigen, verzweigten oder cyclisch gesättigten Kohlenwasserstoffrest aus einem bis zehn Kohlenstoffatomen. Als Halogensubstituent sind die Elemente Fluor, Chlor, Brom und Iod einsetzbar. Die Kohlenstoffatome der Alkylseitenkette Y und der Alkoxykette können asymmetrisch substituiert sein.

Dabei betrifft die Erfindung Verbindungen des einen oder des anderen Enantiomers sowie einer racemischen Mischung oder Mischungen der beiden Antipoden in unterschiedlichen Verhältnissen. Weiterhin können Verbindungen mit zwei Chiralitätszentren in der Alkylseitenkette Y und der Alkoxykette auftreten. In diesem Fall umfaßt die Erfindung sowohl die einzelnen Antipoden für sich, als auch eine Mischung der beiden Enantiomere in unterschiedlichen Verhältnissen, sowie die dazugehörigen Mesoverbindungen.

Die erfindungsgemäßen Verbindungen sind wertvolle Arzneimittel zur Behandlung von Herzrhythmusstörungen unterschiedlichster Genese und zur Verhinderung des arrhythmisch bedingten, plötzlichen Herztods und können daher als Antiarrhythmika Verwendung finden. Beispiele arrhythmischer Störungen des Herzens sind supraventrikuläre Rhythmusstörungen wie etwa Vorhof-Tachycardien Vorhof-Flattern oder paroxysmale supraventriculäre Rhythmusstörungen oder ventrikuläre Rhythmusstörungen wie ventrikuläre Extrasystolen, insbesondere aber lebensbedrohende ventrikuläre Tachykardien oder das besonders gefährliche Kammerflimmern. Sie eignen sich insbesondere für solche Fälle wo Arrhythmien Folge einer Verengung eines Koronargefäßes sind wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztods geeignet. Weitere Krankheitsbilder, wo derartige Rhythmusstörungen und/oder der plötzliche, arrhythmisch bedingte Herztod eine Rolle spielen sind beispielsweise die Herzinsuffizienz oder die Herzhypertrophie als Folge eines chronisch erhöhten Blutdrucks.

Darüberhinaus können die Verbindungen eine verminderte Kontraktilität des Herzens positiv beeinflussen. Hierbei kann es sich um ein krankheitsbedingtes Nachlassen der Herzkontraktilität handeln wie beispielsweise bei Herzinsuffizienz aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch cardioplegische Lösungen erforderlich machen.

Als Versuchstiere zum Nachweis solcher Wirkungen eignen sich zum Beispiel Mäuse, Ratten, Meerschweinchen, Kaninchen, Hunde, Affen oder Schweine. Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Die Erfindung betrifft ferner ein Verfahren zur Herstellung der Verbindungen I, dadurch gekennzeichnet, daß man
(a) ein aromatisches Sulfonamid der Formel II 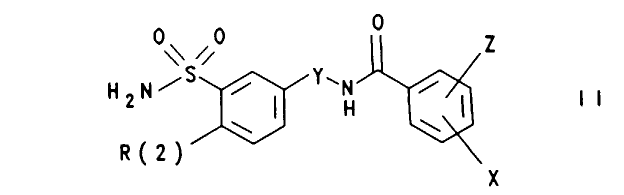 oder dessen Salz der Formel III 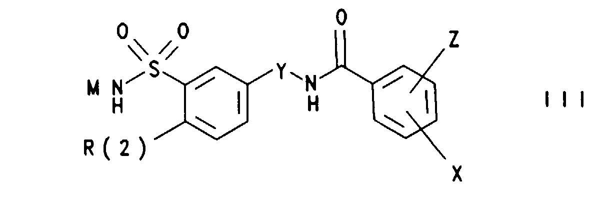 mit einem R(1)-substituierten Isocyanat der Formel IV

   R(1)-N=C=O IV

   zu einem substituierten Benzolsulfonylharnstoff I a umsetzt.
   Als Kationen M in den Salzen der Formel III kommen Alkali- und Erdalkali-Ammonium- sowie Tetraalkylammoniumionen in Betracht. Äquivalent zu den R(1)-substituierten Isocyanaten IV kann man R(1)-substituierte Carbamidsäureester, R(1)-substituierte Carbamidsäurehalogenide oder R(1)-substituierte Harnstoffe einsetzen.
(b) Einen unsubstituierten Benzolsulfonylharnstoff I a [R(1) = H] kann man durch Umsetzung eines aromatischen Benzolsulfonamids der Formel II oder dessen Salzes III mit Trialkylsilylisocyanat oder Siliciumtetraisocynat und Hydrolyse der primären siliciumsubstituierten Benzolsulfonylharnstoffe herstellen. Weiterhin ist es möglich, ein Benzolsulfonamid II oder dessen Salz III durch Umsetzung mit einem Halogencyan und Hydrolyse des primär entstehenden N-Cyanosulfonamids mit Mineralsäuren bei Temperaturen von 0°C bis 100°C darzustellen.
(c) Ein Benzolsulfonylharnstoff I a läßt sich aus einem aromatischen Benzolsulfonamid II oder dessen Salze III mit einem R(1)-substituierten Trichloracetamid der Formel V

   Cl₃C-C(=O)-NHR(1) V

   in Gegenwart einer Base in einem inerten Lösungsmittel nach Synthesis 1987, 734 - 735 bei Temperaturen von 25°C bis 150°C darstellen.
   Als Basen eignen sich zum Beispiel Alkalimetall- oder Erdalkalimetallhydroxide,-hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltrimaid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(d) Ein Benzolsulfonylthioharnstoff I b 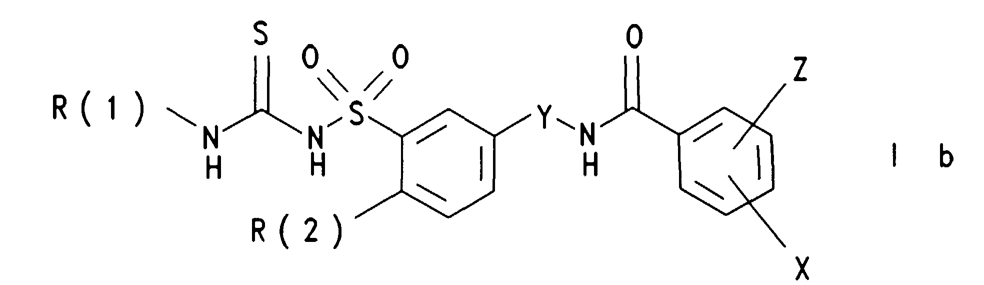 wird aus einem Benzolsulfonamid II oder dessen Salz III und einem R(1)-substituierte Thioisocyanaten VI

   R(1)-N=C=S VI

   dargestellt. Einen unsubstituierten Benzolsulfonylthioharnstoff I b [R(1) = H] kann man durch Umsetzung eines aromatischen Benzolsulfonamids II oder dessen Salzes III mit Trimethylsilylisothiocyanat oder Siliciumtetraisothiocyanat und Hydrolyse des primär entstehenden siliciumsubstituierten Benzolsulfonylharnstoffs herstellen. Weiterhin ist es möglich, ein aromatisches Benzolsulfonamid II oder dessen Salz III mit Benzoylisothiocyanat umzusetzen und den intermediären benzoylsubstituierten Benzolsulfonylthioharnstoff mit wäßriger Mineralsäure zu I b [R(1) = H] umzusetzen. Ähnliche Verfahren sind beschrieben in J. Med. Chem. 1992, 35, 1137-1144.
(e) Einen substituierten Benzolsulfonylharnstoff der Formel I a kann man durch Umwandlungsreaktion aus einem Benzolsulfonylthioharnstoff der Struktur I b darstellen. Der Ersatz des Schwefelatoms durch ein Sauerstoffatom in den entsprechend substituierten Benzolsulfonylthioharnstoffen kann man beispielsweise mit Hilfe von Oxiden oder Salzen von Schwermetallen oder durch auch durch Anwendung von Oxidationsmitteln wie Wasserstoffperoxid, Natriumperoxid oder salpetriger Säure ausgeführt werden. Thioharnstoffe können auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Benzolsulfonylharnstoffe überführt werden. Isothioharnstoffe verhalten sich bei der Entschwefelung wie Thioharnstoffe und können demzufolge ebenso als Ausgangsstoffe für diese Reaktionen dienen.
(f) Einen Benzolsulfonylharnstoff I a kann man aus einem Benzolsulfonylhalogenid der Formel VII 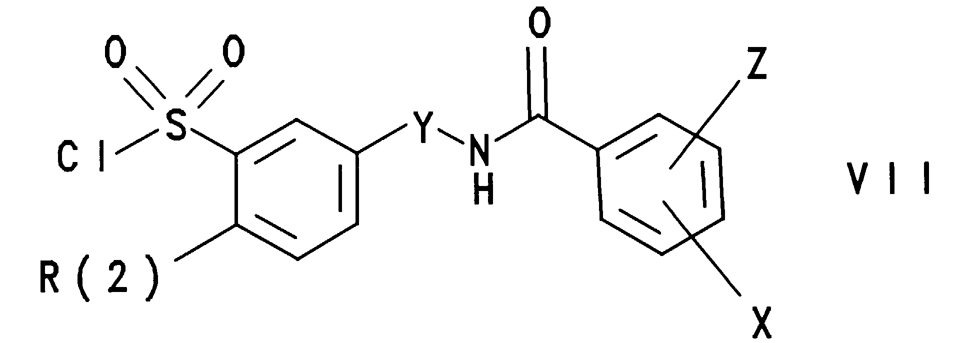 mit einem R(1)-substituierten Harnstoff oder einem R(1)-substituierten Bis(trialkylsilyl)harnstoff herstellen. Die Trialkylsilylschutzgruppe kann aus dem resultierenden (Trialkylsilyl)benzolsulfonylharnstoff nach Standardmethoden entfernt werden. Weiterhin kann man die Sulfonsäurechloride VII mit Parabansäuren zu Benzolsulfonylparabansäuren umsetzen, deren Hydrolyse mit Mineralsäuren die entsprechenden Benzolsulfonylharnstoffe I a liefert.
(g) Ein Benzolsulfonylharnstoff I a läßt sich durch Umsetzung eines Amins der Formel R(1)-NH2 mit einem Benzolsulfonylisocyanat der Formel VIII 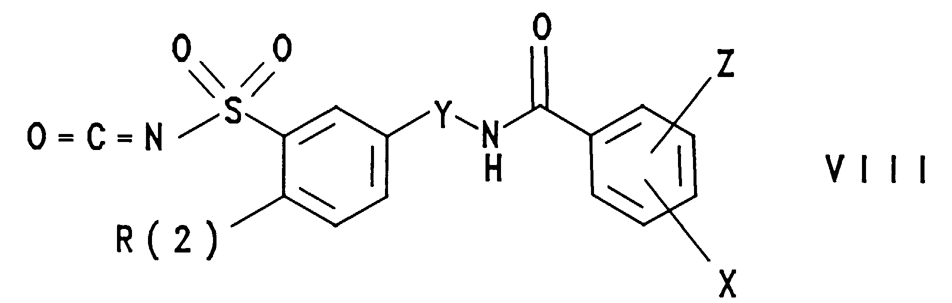 herstellen. Ebenso können Amine R(1)-NH2 mit Benzolsulfonylcarbamidsäureestern, -carbamidsäurehalogeniden oder Benzolsulfonylharnstoffen I a [mit R(1) = H] zu den Verbindungen I a umgesetzt werden.
(h) Ein Benzolsulfonylthioharnstoff I b läßt sich durch Umsetzung eines Amins der Formel R(1)-NH₂ mit einem Benzolsulfonylisothiocyanat der Formel IX 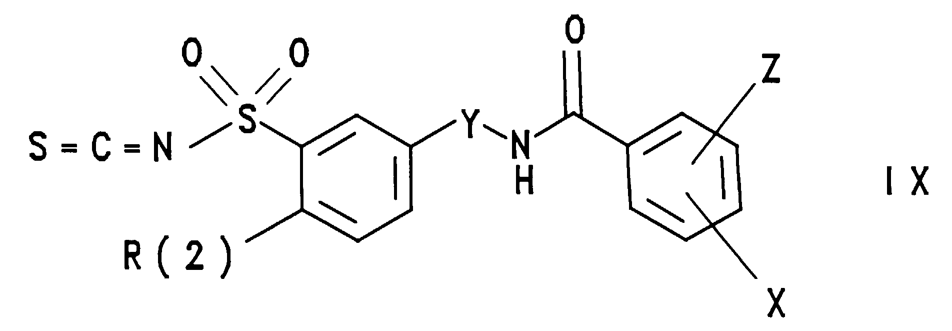 herstellen. Ebenso können Amine R(1)-NH₂ mit einem Benzolsulfonylcarbamidsäurethioester oder einem -carbamidsäurethiohalogenid zur Verbindung I b umgesetzt werden.
(i) Entsprechend substituierte Benzolsulfenyl oder -sulfinylharnstoffe lassen sich mit Oxidationsmitteln wie Wasserstoffperoxid, Natriumperoxid oder salpetriger Säure zu Benzolsulfonylharnstoffen I a oxidieren.

Die Verbindungen I sowie deren physiologisch unbedenkliche Salze sind wertvolle Therapeutika, die sich nicht nur als Antiarrhythmika, sondern auch zur Prophylaxe bei Störungen des cardiovaskulären Systems, Herzinsuffizienz, Herztranplantation oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remmington's Pharmaceutical Science, 17. Auflage, 1985, Seiten 14 - 18 Verbindungen der Formel XI, 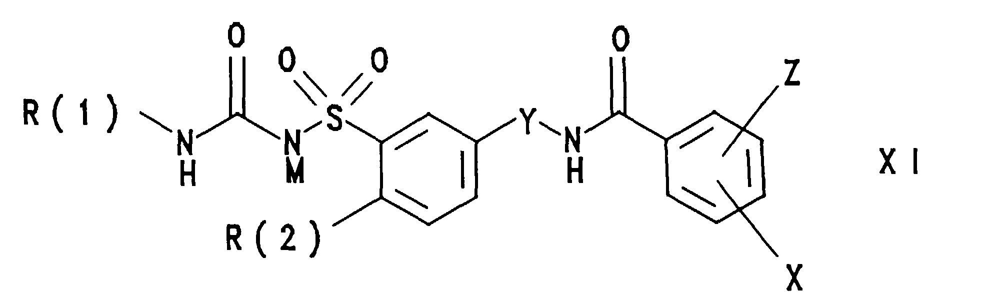 die sich aus nicht toxischen organischen und anorganischen Basen und substituierten Benzolsulfonylharnstoffen I darstellen lassen. Bevorzugt werden hierbei Salze in denen M in der Formel XI Natrium-, Kalium-, Rubidium, Calcium-, Magnesium-, Ammoniumionen sind, sowie die Säureadditionsprodukte basischer Aminosäuren, wie zum Beispiel Lysin oder Arginin sein können.

Die Ausgangsverbindungen für die erwähnten Syntheseverfahren der Benzolsulfonylharnstoffe I werden nach bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentveröffentlichungen) beschrieben sind, und zwar unter Reaktionsbedingungen, die für die genannten Umsetzungen bekannt und geeignet sind. Dabei kann man auch von bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

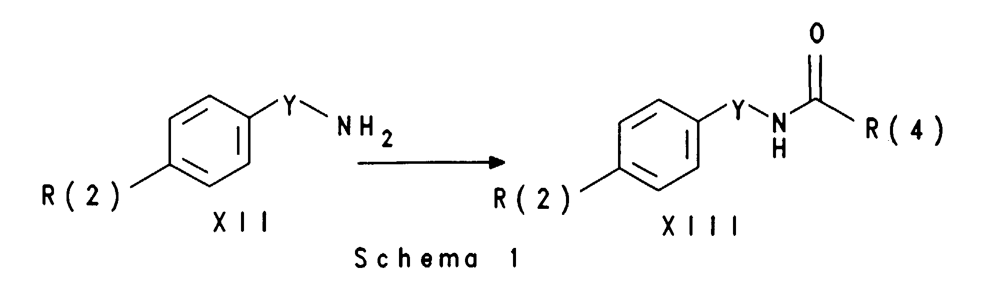

So kann man geeignet substituierte Amine der allgemeinen Formel XII nach Schema 1 acylieren und einer Halogensulfonierung unterwerfen. Als acylierende Mittel zur Acylierung von Aminogruppen eignen sich zweckmäßig die Alkylester, Halogenide (zum Beispiel Chloride oder Bromide) oder Anhydride von Carbonsäuren der Formel R(4)-COB. R(4) steht hierbei für einen Trihalogenmethylrest, einen (C₁-C₄)-Alkylrest oder ein Benzoesäurederivat. Das Benzoesäurederivat kann hierbei unsubstituiert oder durch ein oder zwei gleiche oder verschiedene Reste X, Z substituiert sein. Ein möglicher Substituent X steht für Wasserstoff, (C₁-C₄)-Alkyl oder Halogen, ein Substituent Z für Wasserstoff, Halogen, (C₁-C₄)-Alkyl, (C₁-C₄)-Alkoxy oder Nitro. B ist eine Fluchtgruppe wie zum Beispiel Halogenid, (C₁-C₄)-Alkoxy, Trihalogenacetat, (C₁-C₄)-Carboxylat. Beispiele hierfür sind Acetanhydrid, Trihalogenacetanhydrid, Acetylhalogenid, Trihalogenacetylhalogenid, Propionylchlorid, Isobutyrylbromid und -chlorid, Ameisensäure/Essigsäureanhydrid, Benzoylchlorid, 5-Chlor-2-methoxybenzoesäure-chlorid oder -anhydrid sowie -(C₁-C₄)-alkylester oder 2,5-Difluor-benzoesaürechlorid. Die Synthesen der Verbindung XIII werden unter Zusatz einer tertiären Base wie zum Beispiel Pyridin oder Trialkylamine in Gegenwart oder Abwesenheit eines inerten Lösungsmittels durchgeführt, wobei auch ein Katalysator, zum Beispiel Dimethylaminopyridin, zugegen sein kann. Die Umsetzung kann bei Temperaturen von etwa 0°C bis 160°C, vorzugsweise von 20 bis 150°C erreicht werden. Die Acylgruppe der Amine XII kann sowohl eine Schutzgruppe, als auch, im Fall der Benzoesäurederivate, Teil der Verbindung I sein. Als inerte Lösungsmittel eignen sich Ether wie Tetrahydrofuran, Dioxan, Glykolether wie Ethylenglykolmonomethyl- oder monoethylether (Methylglykol oder Ethylglykol), Ethylenglykoldimethylether (Diglyme), Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Phosphorsäurehexamethyltriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser Lösungsmittel untereinander. 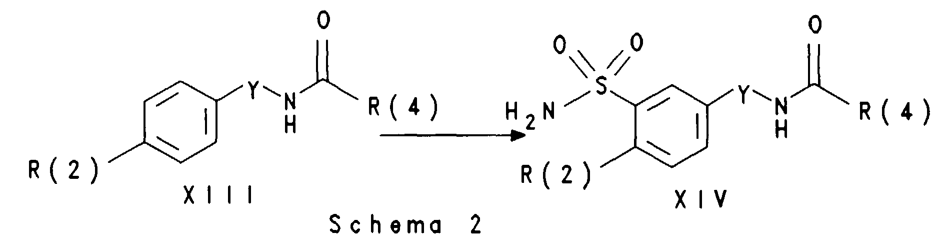 Die nach Schema 1 acylierten Amine XIII können in bekannter Weise nach Schema 2 in die Sulfonamide XIV überführt werden. Die Sulfonamide XIV werden nach bekannten Methoden hergestellt und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Synthesen können gewünschtenfalls in einem, zwei oder mehreren Schritten vollzogen werden. Insbesondere sind Verfahren bevorzugt, in denen das acylierte Amin XII durch elektrophile Reagenzien in An- oder Abwesenheit von inerten Lösungsmitteln bei Temperaturen von -10°C bis 120°C, vorzugsweise von 0°C bis 100°C in aromatische Sulfonsäuren sowie deren Derivate, wie Sulfonsäurehalogenide, überführt wird. Beispielsweise können Sulfonierungen mit Schwefelsäuren oder Oleum, Halogensulfonierungen mit Halogensulfonsäuren, Reaktionen mit Sulfurylhalogeniden in Gegenwart von wasserfreien Metallhalogeniden oder Thionylhalogeniden in Gegenwart von wasserfreien Metallhalogeniden mit anschließenden, in bekannter Weise durchgeführten, Oxidationen zu aromatischen Sulfonsäurechloriden, durchgeführt werden. Sind Sulfonsäuren die primären Reaktionsprodukte, so können diese entweder direkt oder durch Behandlung mit tertiären Aminen, wie zum Beispiel Pyridin oder Trialkylaminen oder mit Alkali- oder Erdalkalihydroxiden oder Reagenzien, die diese basischen Verbindungen in situ bilden, in bekannter Weise durch Säurehalogenide wie zum Beispiel Phosphortrihalogenid, Phosphorpentahalogenid, Phosphoroxychlorid, Thionylhalogenid, Oxalylhalogenid, in Sulfonsäurehalogenide überführt werden. Die Überführung der Sulfonsäurederivate in Sulfonamide erfolgt in literaturbekannter Weise, vorzugsweise werden Sulfonsäurechloride in inerten Lösungsmitteln bei Temperaturen von 0°C bis 100°C mit wäßrigem Ammoniak umgesetzt. Weiterhin kann man aromatische Sulfonamide nach literaturbeschriebenen Verfahren aus den nach Schema 1 hergestellten acylierten Aminen der Formel XIII durch Umsetzungen mit alkali- oder erdalkalimetallorganischen Reagenzien in inerten Lösungsmitteln und unter Inertgasatmosphäre bei Temperaturen von -100°C bis 50°C, vorzugsweise -100°C bis 30°C, mit Schwefeldioxid und anschließender thermischer Behandlung mit Amidosulfonsäure synthetisieren.

Fungiert die Acylgruppe als Schutzgruppe für das Amin XIII dann läßt sich diese nach Darstellung des Sulfonamids XIV mit Säuren oder Basen abspalten. Durch Spaltung mit wässrigen Säuren oder Säuren in inerten Lösungsmitteln kann das zugehörige Säureadditionssalz entstehen. Für diese Umsetzung kommen zum Beispiel Schwefelsäure, Salpetersäure, Halogenwasserstoffsäuren, wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, zum Beispiel Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Phenylessigsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure in Betracht. Die Spaltung des acylierten Amins der Formel XIII mit Basen kann in wäßrigen oder inerten Lösungsmitteln erfolgen. Als Basen eignen sich zum Beispiel Alkalimetall- oder Erdalkalimetallhydroxide, -hydride, -amide oder auch -alkoholate, wie Natriumhydroxid, Kaliumhydroxid, Calciumhydroxyd, Natriumhydrid, Kaliumhydrid, Calciumhydrid, Natriumamid, Kaliumamid, Natriummethylat, Natriumethanolat, Kaliummethylat oder Kaliumethanolat.

Aus den so hergestellten sulfonamidsubstiuierten Aminen oder deren Säureadditionsverbindungen, werden wie oben erwähnt die aromatischen Benzolsulfonamide der Formel III hergestellt. Je nach der Natur der Glieder R(1),R(2), R(3), X, Y und Z wird in einzelnen Fällen das eine oder andere der genannten Verfahren für die Herstellung der Verbindungen I ungeeignet sein oder zumindest Vorkehrungen zum Schutz aktiver Gruppen notwendig machen. Derartige, verhältnismäßig selten auftretende Fälle können vom Fachmann unschwer erkannt werden, und es bereitet keine Schwierigkeiten, in solchen Fällen einen anderen der beschriebenen Synthesewege erfolgreich anzuwenden.

Die Verbindungen I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomere, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäuren, Mandelsäuren, Äpfelsäure oder Milchsäure. Carbinole können ferner mit Hilfe chiraler Acylierungsreagenzien, zum Beispiel R- oder S-α-Methylbenzylisocyanat amidiert und dann getrennt werden. Die verschiedenen Formen der Diastereomeren können in an sich bekannter Weise, zum Beispiel durch fraktionierte Kristallisation, getrennt, und die Enantiomeren der Formel I können in bekannter Weise aus den Diastereomeren in Freiheit gesetzt werden. Enantiomerentrennung gelingt ferner durch Chromatographie an optisch-aktiven Trägermaterialien.

Die erfindungsgemäßen Verbindungen I und ihre physiologisch unbedenklichen Salze können zur Herstellung pharmazeutischer Zubereitungen verwendet werden, insbesondere auf nichtchemischen Weg. Hierbei können sie zusammen mit mindestens einem festen oder flüssigen Träger oder Hilfsstoff allein oder in Kombination mit anderen Herz-Kreislauf-aktiven Arzneimitteln, wie etwa Calcium-Antagonisten, NO-Donatoren oder ACE-Hemmern in eine geeignet Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den Verbindungen I nicht reagieren, beispielsweise Wasser, pflanzliche Öle, Benzylalkohole, Polyethylenglykole, Glycerintricacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen wie Ethanol oder Isopropanol, Acetonitril, DMF, Dimethylacetamid, 1,2-Propandiol oder deren Gemische untereinander oder mit Wasser) oder Puder. Die Verbindungen I können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht, die Stabilisierungs- und/oder Netzmittel, Emulgatoren, Salze und/oder Hilfsstoffe wie Gleit-, Konservierungsmittel, Salze zur Beeinflussung des osmotischen Druckes, Puffersubstanzen, Farb- und Geschmacks- und/oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen I notwendig sind, hängen davon ab ob akut oder prophylaktisch therapiert wird. Normalerweise kommt man mit einem Dosisbereich von etwa mindestens 0.1, vorzugsweise mindestens 1 mg bis höchstens 100, vorzugsweise höchstens 10 mg pro kg und Tag aus, wenn Prophylaxe betrieben wird. Bevorzugt ist ein Dosisbereich von 1 bis 10 mg pro kg und Tag, bezogen auf einen Erwachsenen von etwa 75 kg Gewicht. Die Dosis kann dabei als orale oder parenterale Einzeldosis oder aber in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Eine bevorzugte Dosisbereich in kritischen Situationen kann dann 10 bis 100 mg betragen und beispielsweise als intravenöse Dauerinfusion verabreicht werden. Erfindungsgemäß können außer den in den Ausführungsbeispielen beschriebenen Verbindungen auch die in der folgenden Tabelle zusammengestellten Verbindungen I erhalten werden:
(1) 2-Methoxy-5-chlor-{N-2-[-3-sulfonylamino-N-(methylaminocarbonyl)-4-methoxyethoxyphenyl]-ethyl}-benzamid
(2) 2-Methoxy-5-fluor-{N-2-[-3-sulfonylamino-N-(methylaminocarbonyl)-4-methoxyethoxyphenyl]-ethyl}-benzamid
(3) 2-Methoxy-5-fluor-{N-2-[-3-sulfonylamino-N-(methylaminothiocarbonyl)-4-methoxyethoxyphenyl]-ethyl}-benzamid

### Beispiel 1

2-Methoxy-5-chlor-{N-2-[-3-sulfonylamino-N-(methylaminothiocarbonyl)-4-methoxyethoxyphenyl]-ethyl}-benzamid 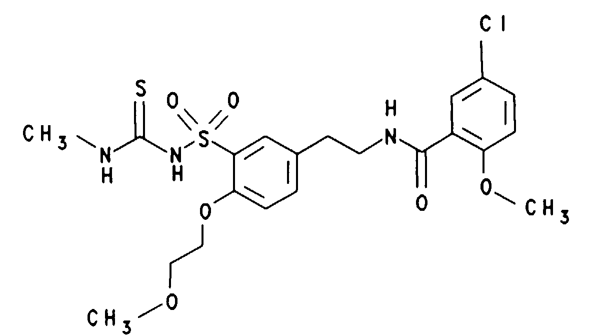 670 mg 2-Methoxy-5-chlor-{N-2-[-3-sulfonylamino-4-methoxyethoxyphenyl]-ethyl}-benzamid werden in 10 ml absolutem DMF gelöst und mit 70 mg 60%igem NaH versetzt. Man rührt 20 min bei Raumtemperatur und tropft 1.6 ml einer 1-molaren Methylisothiocyanatlösung in DMF zu. Man erhitzt 1.5 h auf 80°C und tropft die Reaktionslösung nach Abkühlen auf 100 ml 1 N Salzsäure. Man extrahiert mit Essigester, trocknet und entfernt das Lösungsmittel im Vakuum. Der erhaltene Feststoff wird in wenig Ethanol heiß gelöst und mit Wasser ausgefällt.
Ausbeute 720 mg, Smp. 134°C.

### Beispiel 2

2-Methoxy-5-chlor-{N-2-[-3-sulfonylamino-N-(methylaminothiocarbonyl)-4-methoxyethoxyethoxyphenyl]-ethyl}-benzamid 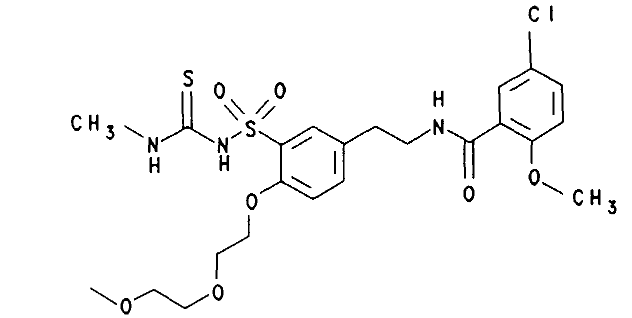 390 mg 2-Methoxy-5-chlor-{N-2-[-3-sulfonylamino-4-methoxyethoxyethoxyphenyl]-ethyl}-benzamid werden in 6 ml DMF gelöst und mit 35 mg 60%igem NaH versetzt. Man rührt 20 min bei Raumtemperatur und tropft 0.8 ml einer 1-molaren Methylisothiocyanatlösung in DMF zu. Man erhitzt 1.5 h auf 80°C und tropft die Reaktionslösung nach Abkühlen auf 50 ml 1 N Salzsäure. Man extrahiert mit Essigester, trocknet und entfernt das Lösungsmittel im Vakuum.
Smp. 108°C.

### Pharmakologische Daten:

Mit den folgenden Modellen können die therapeutischen Eigenschaften der Verbindungen I nahegelegt werden:

### (1) Aktionspotentialdauer am Papillarmuskel des Meerschweinchens:

### (a) Einleitung

ATP-Mangelzustände wie sie während einer Ischämie in der Herzmuskelzelle beobachtet werden führen zu einer Verkürzung der Aktionspotentialdauer. Sie gelten als einer der Ursachen für sogenannte Reentry-Arrhythmien, die den plötzlichen Herztod verursachen können. Die Öffnung von ATP-sensitiven K-Kanälen durch das Absinken von ATP gilt hierfür als ursächlich.

### (b) Methode

Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCl, 0.048% KCl, 0.024% CaCl₂, 0.02% NaHCO₃ und 0.1% Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2·10⁻⁵ Mol pro Liter zugesetzt. Das Aktionspotential wird mit einem Amplifier von Hugo Sachs verstärkt und am Oszilloskop dargestellt. Die Dauer des Aktionspotentials wird bei einem Repolarisierungsgrad von 95% (APD95) bestimmt. Aktionspotential-Verkürzungen werden entweder durch Zugabe einer 1 µM starken Lösung des Kaliumkanalöffners Hoe 234 [J. Kaiser, H. Gögelein, Naunyn-Schmiedebergs Arch. Pharm. 1991, 343, R(59)] oder durch Zugabe von 2-Desoxyglucose hervorgerufen. Der aktionspotentialverkürzende Effekt dieser Substanzen wurde durch die gleichzeitige Gabe der Testsubstanzen verhindert oder verringert. Testsubstanzen wurden als Stammlösungen in Propandiol der Badlösung zugesetzt. Die angegebenen Werte beziehen sich auf Messungen 30 min nach der Zugabe. Glibenclamid diente in diesen Messungen als Standard. Die Testkonzentration beträgt in allen Fällen 2 x 10⁻⁶ M

### (c) Resultate:

Folgende Werte wurden gemessen:.

| Beispiel Nr. | APD95-Anfang [ms] | APD95-30 min [ms] |
|---|---|---|
| 1 | 160 ± 13 | 150 ± 14 |

## Patentansprüche

1. Substituierte Benzolsulfonylharnstoffe und -thioharnstoffe der Formel I, 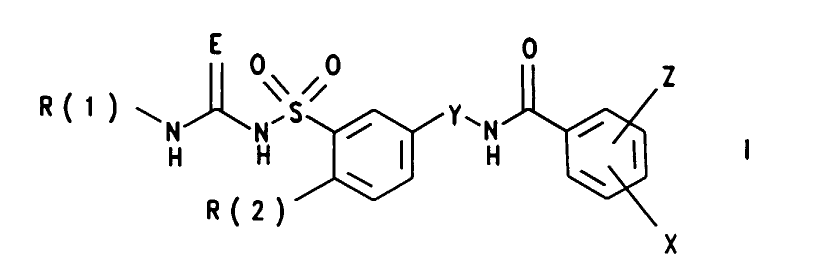 worin bedeuten:
R(1) Wasserstoff, Methyl oder Trifluormethyl;
R(2) Alkoxy mit 4, 5, 6, 7, 8, 9 oder 10 C-Atomen, wobei 1 bis 6 Kohlenstoffatome gegen die Heteroatome O, S oder NH ausgetauscht sind;
E Sauerstoff oder Schwefel;
Y -[CR(3)₂]₁₋₄;
R(3) Wasserstoff oder Alkyl mit 1 oder 2 C-Atomen;
X Wasserstoff, Halogen oder Alkyl mit 1, 2, 3, 4, 5 oder 6 C-Atomen;
Z Nitro, Halogen, Alkoxy mit 1, 2, 3 oder 4 C-Atomen oder Alkyl mit 1, 2, 3 oder 4 C-Atomen,
sowie ihre pharmazeutisch verträglichen Salze.

2. Verbindung der Formel I nach Anspruch 1, dadurch gekennzeichnet, daß darin bedeuten:
R(1) Wasserstoff, Methyl oder Trifluormethyl;
R(2) Alkoxy mit 4, 5, 6, 7, 8, 9 oder 10 C-Atomen, worin ein bis sechs Kohlenstoffatome gegen die Heteroatome O, NH oder S ausgetauscht sind;
E Sauerstoff oder Schwefel;
Y ein gerader substituierter oder unsubstituierter Kohlenwasserstoffrest der Formel: -[CR(3)₂]₁₋₄;
R(3) Wasserstoff oder Alkyl mit 1 oder 2 C-Atomen;
X Wasserstoff, Chlor, Fluor oder Alkyl mit 1, 2, 3 oder 4 C-Atomen;
Z Nitro, Fluor, Chlor, Alkyl mit 1, 2, 3 oder 4 C-Atomen oder Alkoxy mit 1, 2, 3 oder 4 C-Atomen;
sowie ihre pharmazeutisch verträglichen Salze.

3. Verbindung der Formel I nach mindestens einem der Ansprüche 1 und 2, dadurch gekennzeichnet, daß darin bedeuten:
R(1) Wasserstoff oder Methyl;
R(2) Alkoxy mit 4, 5, 6, 7, 8, 9 oder 10 C-Atomen, bei der 1 bis 6 Kohlenstoffatome gegen die Heteroatome O, S oder NH ausgetauscht sind;
E Sauerstoff oder Schwefel;
Y -[CR(3)₂]₁₋₄;
R(3) Wasserstoff oder Alkyl mit 1 oder 2 C-Atomen;
X Wasserstoff, Fluor, Chlor oder Alkyl mit 1, 2, 3 oder 4 C-Atomen;
Z Chlor, Fluor, Alkyl mit 1, 2, 3 oder 4 C-Atomen oder Alkoxy mit 1, 2, 3 oder 4 C-Atomen;
sowie ihre pharmazeutisch verträgliichen Salze.

4. Verbindung der Formel I nach mindestens einem der Ansprüche 1 bis 3, dadurch gekennzeichnet, daß darin bedeuten:
R(1) Wasserstoff oder Methyl;
R(2) Methoxyethoxy oder Methoxyethoxyethoxy;
E Sauerstoff oder Schwefel;
Y -[CR(3)₂]₂₋₃;
R(3) Wasserstoff oder Methyl;
X Wasserstoff, Fluor, Chlor oder Alkyl mit 1, 2 oder 3-C-Atomen;
Z Fluor, Chlor, Alkyl mit 1, 2 oder 3-C-Atomen oder Alkoxy mit 1, 2 oder 3-C-Atomen;
sowie ihre pharmazeutisch verträglichen Salze.

5. Verbindung der Formel I nach mindestens einem der Ansprüche 1 bis 4, dadurch gekennzeichnet, daß es sich um die Verbindung der Formel 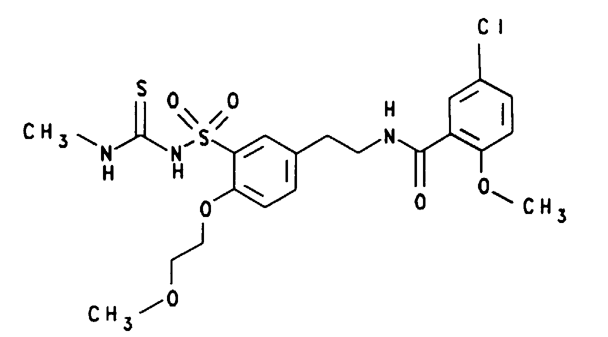 handelt; sowie ihre pharmazeutisch verträglichen Salze.

6. Verbindung der Formel I nach mindestens einem der Ansprüche 1 bis 4, dadurch gekennzeichnet, daß es sich um die Verbindung der Formel 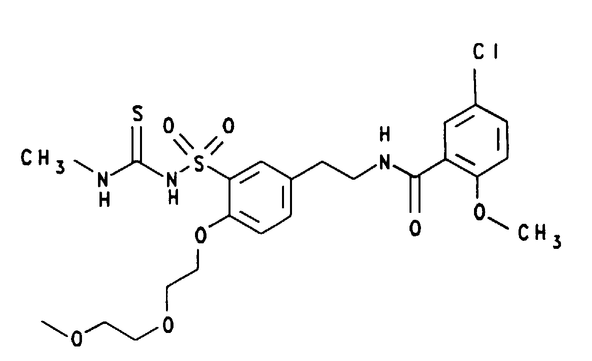 handelt; sowie ihre pharmazeutisch verträglichen Salze.

7. Verfahren zur Herstellung einer Verbindung I nach den Ansprüchen 1 bis 6, dadurch gekennzeichnet, daß man
(a) ein aromatisches Sulfonamid der Formel II 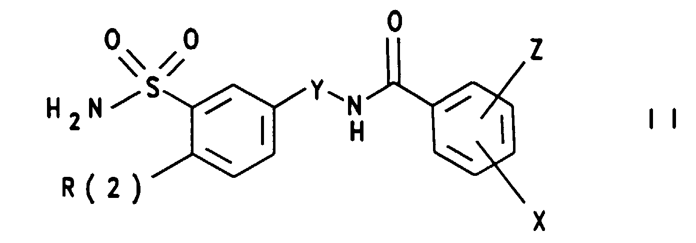 oder dessen Salz der Formel III 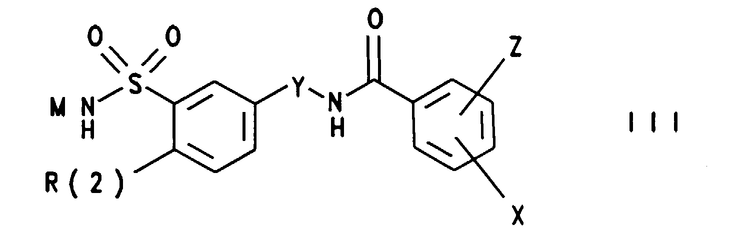 mit einem R(1)-substituierten Isocyanat der Formel IV
R(1)-N=C=O IV
zu einem substituierten Benzolsulfonylharnstoff Ia umsetzt, wobei R(1), R(2), X, Y und Z die in den Ansprüchen 1 bis 6 angegebenen Bedeutungen haben und wobei M ein Alkali-, Erdalkali-, Ammonium- oder Tetraalkylammoniumion ist;
oder daß man
(b) einen unsubstituierten Benzolsulfonylharnstoff I a [R(1) = H] durch Umsetzen eines aromatischen Benzolsulfonamids der Formel II oder dessen Salzes III mit Trialkylsilylisocyanat oder Siliciumtetraisocynat und Hydrolyse der primären siliciumsubstituierten Benzolsulfonylharnstoffe herstellt;
oder daß man
(c) einen Benzolsulfonylharnstoff I a aus einem aromatischen Benzolsulfonamid II oder dessen Salze III mit einem R(1)-substituierten Trichloracetamid der Formel V
Cl₃C-C(=O)-NHR(1) V
in Gegenwart einer Base darstellt;
oder daß man
(d) einen Benzolsulfonylthioharnstoff I b 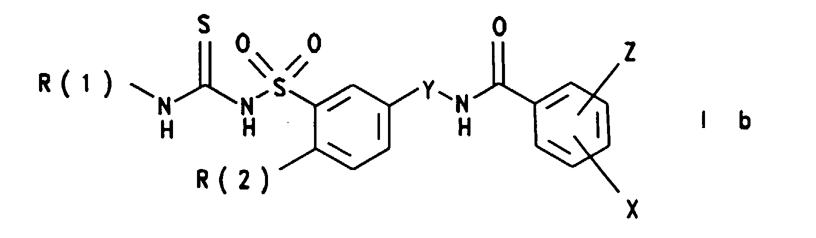 aus einem Benzolsulfonamid II sowie dessen Salz III und einem R(1)-substituierte Thioisocyanat VI
R(1)-N=C=S VI
darstellt;
oder daß man
(e) einen substituierten Benzolsulfonylharnstoff der Formel I a durch Umwandlungsreaktion aus einem Benzolsulfonylthioharnstoff der Struktur I b darstellt;
oder daß man
(f) einen Benzolsulfonylharnstoff I a aus einem Benzolsulfonylhalogenid der Formel VII 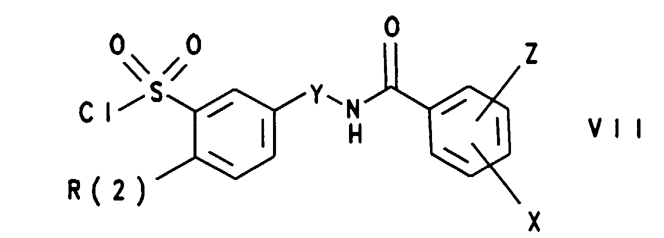 mit einem R(1)-substituierten Harnstoff oder einem R(1)-substituierten Bis(trialkylsilyl)harnstoff herstellt;
oder daß man
(g) einen Benzolsulfonylharnstoff Ia durch Umsetzung eines Amins der Formel R(1)-NH₂ mit einem Benzolsulfonylisocyanat der Formel VIII 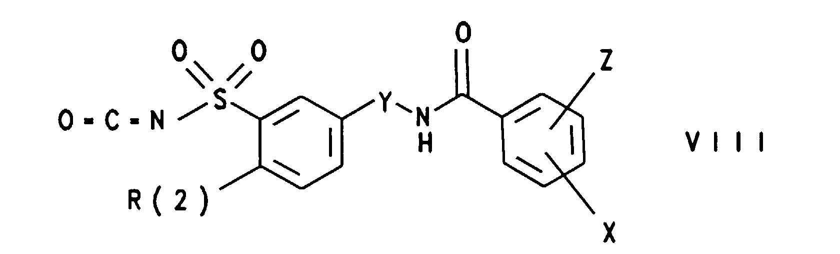 herstellt;
oder daß man
(h) einen Benzolsulfonylthioharnstoff I durch Umsetzung eines Amins der Formel R(1)-NH₂ mit einem Benzolsulfonylisothiocyanat der Formel IX 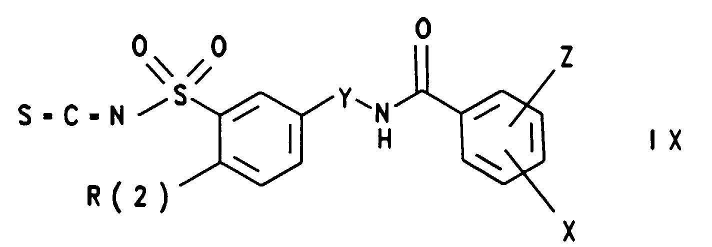 herstellt;
oder daß man
(i) einen substituierten Benzolsulfenyl- oder -sulfinylharnstoff zu einem Benzolsulfonylharnstoff I a oxidiert,
und daß man die Verbindung I gegebenenfalls in das pharmazeutisch verträgliche Salz überführt.

8. Verwendung einer Verbindung der Formel I nach einem oder mehreren der Ansprüche 1 bis 6 oder eines pharmazeutisch verträglichen Salzes davon zur Herstellung eines Medikaments zur Behandlung von Herzrhythmusstörungen.

9. Verwendung einer Verbindung der Formel I nach einem oder mehreren der Ansprüche 1 bis 6 oder eines pharmazeutisch verträglichen Salzes davon zur Herstellung eines Medikaments zur Verhinderung des plötzlichen Herztods.

10. Verwendung einer Verbindung der Formel I nach einem oder mehreren der Ansprüche 1 bis 6 oder eines pharmazeutisch verträglichen Salzes davon zur Herstellung eines Medikaments zur Behandlung von ischämischen Zuständen des Herzens.

11. Verwendung einer Verbindung der Formel I nach einem oder mehreren der Ansprüche 1 bis 6 oder eines pharmazeutisch verträglichen Salzes davon zur Herstellung eines Medikaments zur Behandlung der geschwächten Herzkraft.

12. Verwendung einer Verbindung der Formel I nach einem oder mehreren der Ansprüche 1 bis 6 oder eines pharmazeutisch verträglichen Salzes davon zur Herstellung eines Medikaments zur Verbesserung der Herzfunktion nach Herztransplantation.

13. Heilmittel, gekennzeichnet durch eine wirksame Menge an einer Verbindung der Formel I nach einem oder mehreren der Ansprüche 1 bis 6 oder einem pharmazeutisch verträglichen Salz davon.

## Claims

1. A substituted benzenesulfonylurea or -thiourea of the formula I 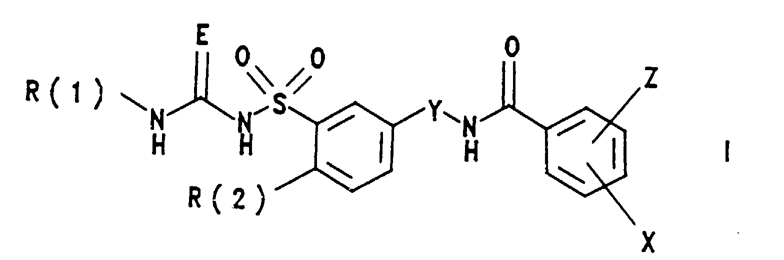 in which:
R(1) is hydrogen, methyl or trifluoromethyl;
R(2) is alkoxy having 4, 5, 6, 7, 8, 9 or 10 carbon atoms, where 1 to 6 carbon atoms are replaced by the heteroatoms O, S or NH;
E is oxygen or sulfur;
Y is - [CR(3)₂]₁₋₄;
R(3) is hydrogen or alkyl having 1 or 2 carbon atoms;
X is hydrogen, halogen or alkyl having 1, 2, 3, 4, 5 or 6 carbon atoms;
Z is nitro, halogen, alkoxy having 1, 2, 3 or 4 carbon atoms or alkyl having 1, 2, 3 or 4 carbon atoms;
or its pharmaceutically tolerable salts.

2. A compound of the formula I as claimed in claim 1, wherein:
R(1) is hydrogen, methyl or trifluoromethyl;
R(2) is alkoxy having 4, 5, 6, 7, 8, 9 or 10 carbon atoms, in which one to six carbon atoms are replaced by the heteroatoms O, NH or S;
E is oxygen or sulfur;
Y is a straight substituted or unsubstituted hydrocarbon radical of the formula:
-[CR(3)₂]₁₋₄;
R(3) is hydrogen or alkyl having 1 or 2 carbon atoms;
X is hydrogen, chlorine, fluorine or alkyl having 1, 2, 3 or 4 carbon atoms;
Z is nitro, fluorine, chlorine, alkyl having 1, 2, 3 or 4 carbon atoms or alkoxy having 1, 2, 3 or 4 carbon atoms;
or its pharmaceutically tolerable salts.

3. A compound of the formula I as claimed in at least one of claims 1 and 2, wherein:
R(1) is hydrogen or methyl;
R(2) is alkoxy having 4, 5, 6, 7, 8, 9 or 10 carbon atoms, in which 1 to 6 carbon atoms are replaced by the heteroatoms O, S or NH;
E is oxygen or sulfur;
Y is -[CR(3)₂]₁₋₄;
R(3) is hydrogen or alkyl having 1 or 2 carbon atoms;
X is hydrogen, fluorine, chlorine or alkyl having 1, 2, 3 or 4 carbon atoms;
Z is chlorine, fluorine, alkyl having 1, 2, 3 or 4 carbon atoms or alkoxy having 1, 2, 3 or 4 carbon atoms;
or its pharmaceutically tolerable salts.

4. A compound of the formula I as claimed in at least one of claims 1 to 3, wherein:
R(1) is hydrogen or methyl;
R(2) is methoxyethoxy or methoxyethoxyethoxy;
E is oxygen or sulfur;
Y is - [CR(3)₂]₂₋₃;
R(3) is hydrogen or methyl;
X is hydrogen, fluorine, chlorine or alkyl having 1, 2 or 3 carbon atoms;
Z is fluorine, chlorine, alkyl having 1, 2 or 3 carbon atoms or alkoxy having 1, 2 or 3 carbon atoms;
or its pharmaceutically tolerable salts.

5. A compound of the formula I as claimed in at least one of claims 1 to 4, which is the compound of the formula 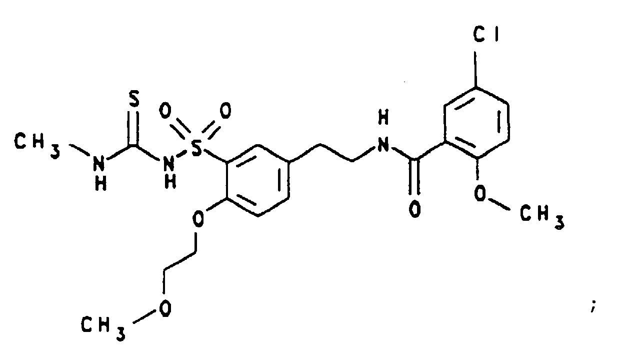 or its pharmaceutically tolerable salts.

6. A compound of the formula I as claimed in at least one of claims 1 to 4, which is the compound of the formula 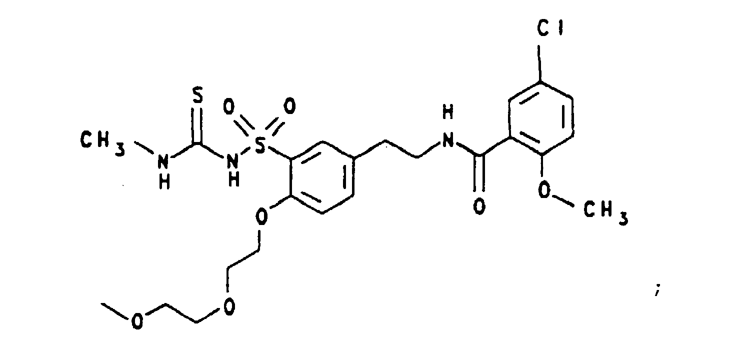 or its pharmaceutically tolerable salts.

7. A process for the preparation of a compound I as claimed in claims 1 to 6, which comprises
(a) reacting an aromatic sulfonamide of the formula II 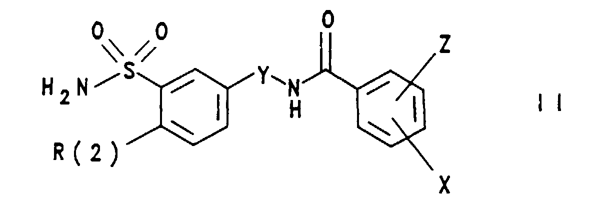 or its salt of the formula III 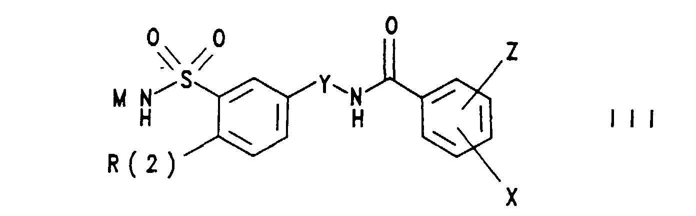 with an R(1)-substituted isocyanate of the formula IV
R(1)-N=C=O IV
to give a substituted benzenesulfonylurea I a, where R(1), R(2), X, Y and Z have the meanings indicated in claims 1 to 6 and where M is an alkali metal, alkaline earth metal, ammonium or tetraalkylammonium ion;
or
(b) preparing an unsubstituted benzenesulfonylurea I a [R(1) = H] by reaction of an aromatic benzenesulfonamide of the formula II or its salt III with trialkylsilyl isocyanate or silicon tetraisocyanate and hydrolysis of the primary silicon-substituted benzenesulfonylureas;
or
(c) preparing a benzenesulfonylurea I a from an aromatic benzenesulfonamide II or its salts III using an R(1)-substituted trichloroacetamide of the formula V
Cl₃C-C(=O)-NHR(1) V
in the presence of a base;
or
(d) preparing a benzenesulfonylthiourea I b 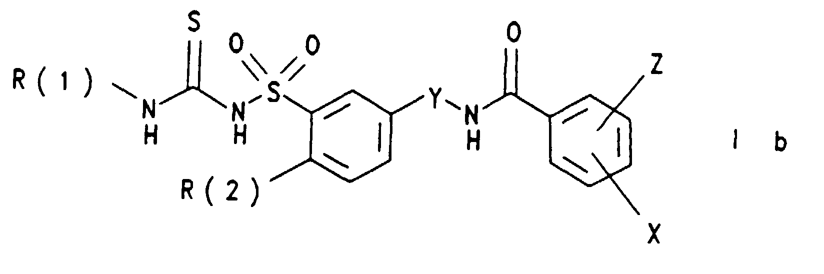 from a benzenesulfonamide II or its salt III and an R(1)-substituted isothiocyanate VI
R(1)-N=C=S VI;
or
(e) preparing a substituted benzenesulfonylurea of the formula I a by conversion reaction from a benzenesulfonylthiourea of the structure I b;
or
(f) preparing a benzenesulfonylurea I a from a benzenesulfonyl halide of the formula VII 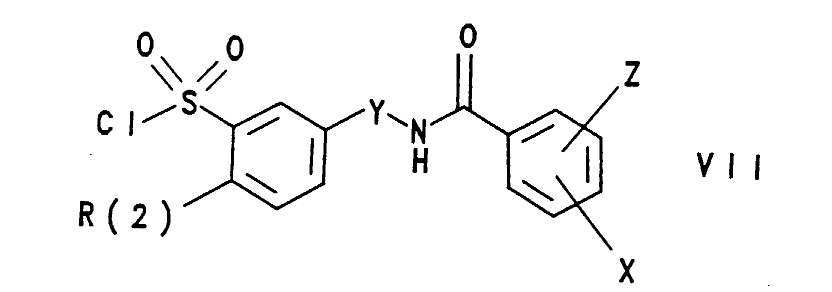 using an R(1)-substituted urea or an R(1)-substituted bis(trialkylsilyl)urea;
or
(g) preparing a benzenesulfonylurea I a by reaction of an amine of the formula R(1)-NH₂ with a benzenesulfonyl isocyanate of the formula VIII 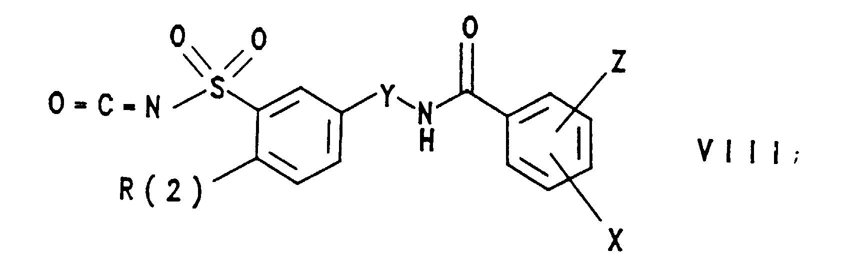 or
(h) preparing a benzenesulfonylthiourea I b by reaction of an amine of the formula R(1)-NH₂ with a benzenesulfonyl isothiocyanate of the formula IX 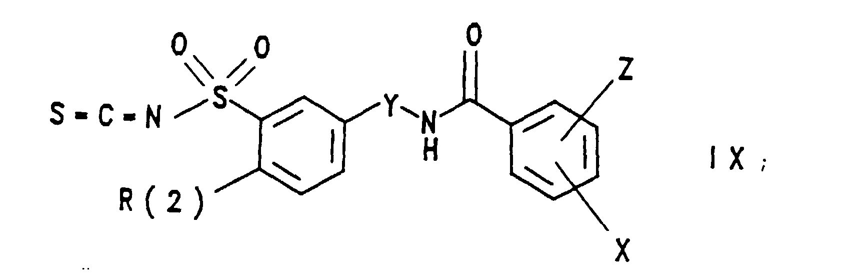 or
(i) oxidizing a substituted benzenesulfenyl- or -sulfinylurea to give a benzenesulfonylurea I a,
and optionally converting the compound I into the pharmaceutically tolerable salt.

8. The use of a compound of the formula I as claimed in one or more of claims 1 to 6 or of a pharmaceutically tolerable salt thereof for the production of a medicament for the treatment of cardiac arrhythmias.

9. The use of a compound of the formula I as claimed in one or more of claims 1 to 6 or of a pharmaceutically tolerable salt thereof for the production of a medicament for the prevention of sudden heart death.

10. The use of a compound of the formula I as claimed in one or more of claims 1 to 6 or of a pharmaceutically tolerable salt thereof for the production of a medicament for the treatment of ischemic conditions of the heart.

11. The use of a compound of the formula I as claimed in one or more of claims 1 to 6 or of a pharmaceutically tolerable salt thereof for the production of a medicament for the treatment of weakened myocardial contractile force.

12. The use of a compound of the formula I as claimed in one or more of claims 1 to 6 or of a pharmaceutically tolerable salt thereof for the production of a medicament for the improvement of heart function after heart transplantation.

13. A medicament comprising an effective amount of a compound of the formula I as claimed in one or more of claims 1 to 6 or of a pharmaceutically tolerable salt thereof.

## Revendications

1. Benzènesulfonylurées et -thio-urées substituées de formule I 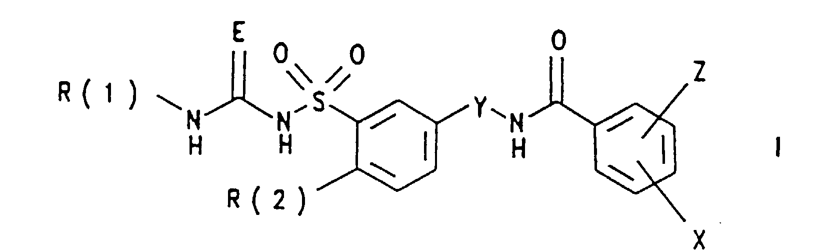 dans laquelle
R(1) représente un atome d'hydrogène ou le groupe méthyle ou trifluorométhyle,
R(2) représente un groupe alcoxy ayant 4, 5, 6, 7, 8, 9 ou 10 atomes de carbone, 1 à 6 atomes de carbone pouvant être remplacés par les hétéroatomes O, S ou par NH,
E représente un atome d'oxygène ou de soufre,
Y représente un groupe -[CR(3)₂]₁₋₄,
R(3) étant un atome d'hydrogène ou un groupe alkyle ayant 1 ou 2 atomes de carbone,
X représente un atome d'hydrogène ou d'halogène ou un groupe alkyle ayant 1, 2, 3, 4, 5 ou 6 atomes de carbone,
Z représente un atome d'halogène ou un groupe nitro, alcoxy ayant 1, 2, 3 ou 4 atomes de carbone ou alkyle ayant 1, 2, 3 ou 4 atomes de carbone,
ainsi que leurs sels pharmaceutiquement acceptables.

2. Composé de formule I selon la revendication 1, caractérisé en ce que dans cette formule:
R(1) représente un atome d'hydrogène ou le groupe méthyle ou trifluorométhyle,
R(2) représente un groupe alcoxy ayant 4, 5, 6, 7, 8, 9 ou 10 atomes de carbone, 1 à 6 atomes de carbone pouvant être remplacés par les hétéroatomes O, S ou par NH,
E représente un atome d'oxygène ou de soufre,
Y représente un radical hydrocarboné à chaîne droite, substitué ou non substitué, de formule -[CR(3)₂]₁₋₄,
R(3) étant un atome d'hydrogène ou un groupe alkyle ayant 1 ou 2 atomes de carbone,
X représente un atome d'hydrogène, de chlore ou de fluor, ou un groupe alkyle ayant 1, 2, 3, ou 4 atomes de carbone,
Z représente un atome de chlore ou de fluor, ou un groupe nitro, alkyle ayant 1, 2, 3 ou 4 atomes de carbone ou alcoxy ayant 1, 2, 3 ou 4 atomes de carbone,
ainsi que ses sels pharmaceutiquement acceptables.

3. Composé de formule I selon au moins l'une des revendications 1 et 2, caractérisé en ce que dans cette formule:
R(1) représente un atome d'hydrogène ou le groupe méthyle,
R(2) représente un groupe alcoxy ayant 4, 5, 6, 7, 8, 9 ou 10 atomes de carbone, 1 à 6 atomes de carbone pouvant être remplacés par les hétéroatomes O, S ou par NH,
E représente un atome d'oxygène ou de soufre,
Y représente un radical -[CR(3)₂]₁₋₄,
R(3) étant un atome d'hydrogène ou un groupe alkyle ayant 1 ou 2 atomes de carbone,
X représente un atome d'hydrogène, de chlore ou de fluor, ou un groupe alkyle ayant 1, 2, 3, ou 4 atomes de carbone,
Z représente un atome de chlore ou de fluor, ou un groupe alkyle ayant 1, 2, 3 ou 4 atomes de carbone ou alcoxy ayant 1, 2, 3 ou 4 atomes de carbone,
ainsi que ses sels pharmaceutiquement acceptables.

4. Composé de formule I selon au moins l'une des revendications 1 à 3, caractérisé en ce que dans cette formule:
R(1) représente un atome d'hydrogène ou le groupe méthyle,
R(2) représente le groupe méthoxyéthoxy ou méthoxyéthoxyéthoxy,
E représente un atome d'oxygène ou de soufre,
Y représente -[CR(3)₂]₂₋₃,
R(3) étant un atome d'hydrogène ou le groupe méthyle,
X représente un atome d'hydrogène, de chlore ou de fluor, ou un groupe alkyle ayant 1, 2 ou 3 atomes de carbone,
Z représente un atome de chlore ou de fluor, ou un groupe alkyle ayant 1, 2 ou 3 atomes de carbone ou alcoxy ayant 1, 2 ou 3 atomes de carbone,
ainsi que ses sels pharmaceutiquement acceptables.

5. Composé de formule I selon au moins l'une des revendications 1 à 4, caractérisé en ce qu'il s'agit du composé de formule: 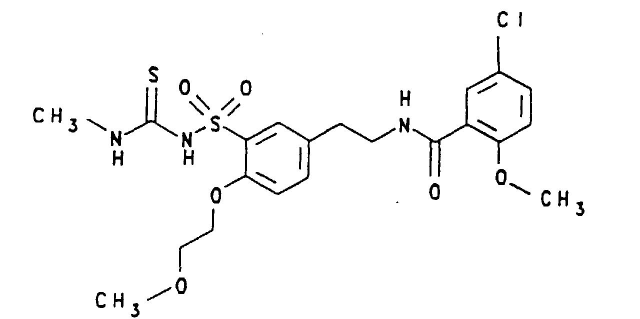 ainsi que ses sels pharmaceutiquement acceptables.

6. Composé de formule I selon au moins l'une des revendications 1 à 4, caractérisé en ce qu'il s'agit du composé de formule: 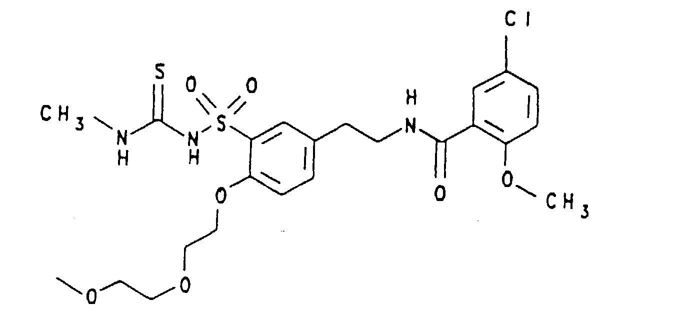 ainsi que ses sels pharmaceutiquement acceptables.

7. Procédé pour la préparation d'un composé I selon les revendications 1 à 6, caractérisé en ce que
(a) on fait réagir un sulfonamide aromatique de formule II 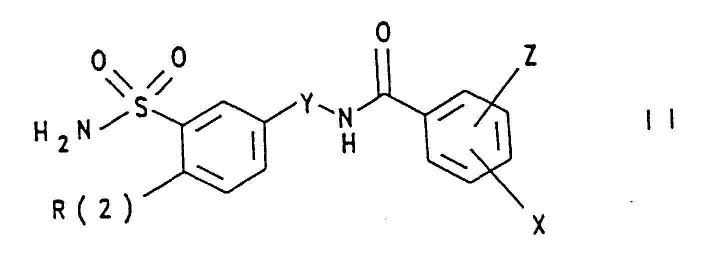 ou un sel de celui-ci, de formule III 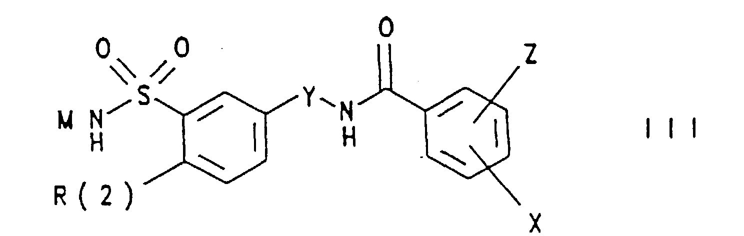 avec un isocyanate substitué par R(1), de formule IV
R(1)-N=C=O IV
pour aboutir à une benzènesulfonylurée substituée Ia, R(1), R(2), X, Y et Z ayant les significations données dans les revendications 1 à 6, et M étant un ion alcalin, alcalino-terreux, ammonium ou tétraalkylammonium; ou en ce que
(b) on prépare une benzènesulfonylurée non substituée Ia [R(1) = H] par la réaction d'un benzènesulfonamide aromatique de formule II ou d'un sel III de celui-ci, avec un trialkylsilylisocyanate ou tétra-isocyanate de silicium, et hydrolyse des benzènesulfonylurées primaires, substituées par le silicium;
ou en ce que
(c) on prépare une benzènesulfonylurée Ia à partir d'un benzènesulfonamide aromatique II ou d'un sel III de celui-ci, et d'un trichloracétamide substitué par le groupe R(1), de formule V
Cl₃C-C(=O)-NHR(1) V
en présence d'une base;
ou en ce que
(d) on prépare une benzènesulfonylthio-urée Ib 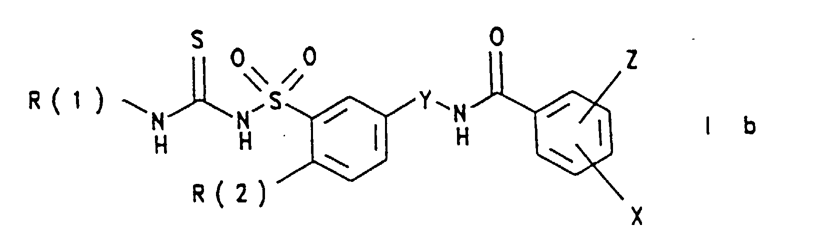 à partir d'un benzènesulfonamide II ainsi qu'un de ses sels III et d'un thio-isocyanate VI substitué par R(1)
R(1)-N=C=S VI
ou en ce que
(e) on prépare une benzènesulfonylurée substituée de formule Ia par une réaction de conversion à partir d'une benzènesulfonylthio-urée de structure Ib;
ou en ce que
(f) on prépare une benzènesulfonylurée Ia à partir d'un halogénure de benzènesulfonyle de formule VII 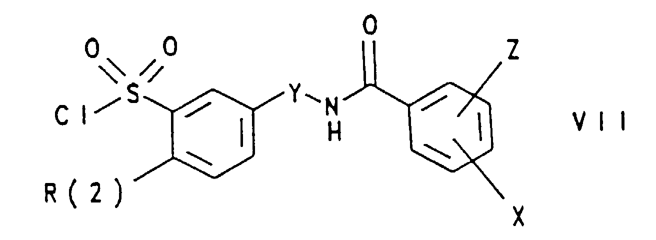 avec une urée substituée par R(1) ou une bis(trialkylsilyl)-urée substituée par R(1);
ou en ce que
(g) on prépare une benzènesulfonylurée Ia par la réaction d'une amine de formule R(1)-NH₂ avec un benzènesulfonylisocyanate de formule VIII 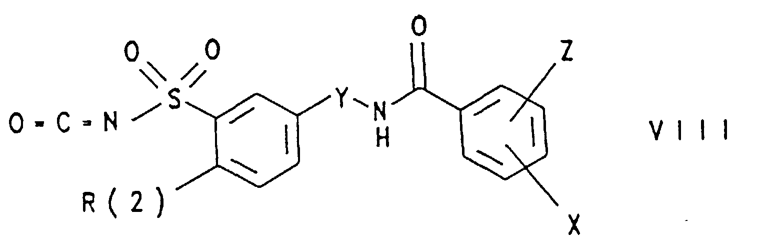 ou en ce que
(h) on prépare une benzènesulfonylthio-urée Ib par la réaction d'une amine de formule R(1)-NH₂ avec un benzène-sulfonylisothiocyanate de formule IX 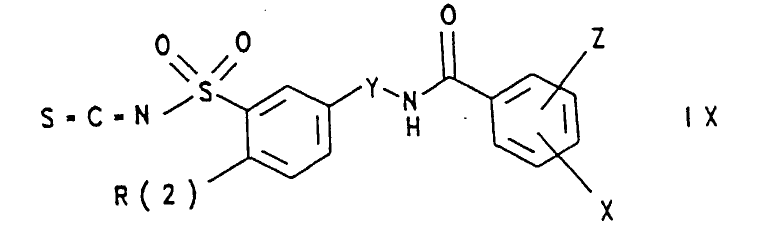 ou en ce que
(i) on oxyde une benzènesulfényl- ou -sulfinylurée substituée en une benzènesulfonylurée Ia;
et en ce que le composé I est éventuellement converti en un sel pharmacologiquement acceptable.

8. Utilisation d'un composé de formule I selon une ou plusieurs des revendications 1 à 6, ou d'un sel pharmaceutiquement acceptable de celui-ci, pour la fabrication d'un médicament destiné au traitement de troubles du rythme cardiaque.

9. Utilisation d'un composé de formule I selon une ou plusieurs des revendications 1 à 6, ou d'un sel pharmaceutiquement acceptable de celui-ci, pour la fabrication d'un médicament destiné à empêcher la mort subite par arrêt cardiaque.

10. Utilisation d'un composé de formule I selon une ou plusieurs des revendications 1 à 6, ou d'un sel pharmaceutiquement acceptable de celui-ci, pour la fabrication d'un médicament destiné au traitement d'états ischémiques du coeur.

11. Utilisation d'un composé de formule I selon une ou plusieurs des revendications 1 à 6, ou d'un sel pharmaceutiquement acceptable de celui-ci, pour la fabrication d'un médicament destiné au traitement de la force cardiaque affaiblie.

12. Utilisation d'un composé de formule I selon une ou plusieurs des revendications 1 à 6, ou d'un sel pharmaceutiquement acceptable de celui-ci, pour la fabrication d'un médicament destiné à l'amélioration de la fonction cardiaque après une greffe du coeur.

13. Médicament, caractérisé par une quantité efficace d'un composé de formule I selon une ou plusieurs des revendications 1 à 6, ou d'un sel pharmaceutiquement acceptable d'un tel composé.
